# FASCICULE DE BREVET EUROPEEN

(11) **EP 2 473 846 B1**
(45) Date de publication et mention de la délivrance du brevet: **13.04.2016**
(21) Numéro de dépôt: 10750103.3
(22) Date de dépôt: 31.08.2010
(51) Int. Cl.: G01N 33/483

(54) **PROCEDE ET DISPOSITIF POUR IDENTIFIER UN PRODUIT SUSCEPTIBLE DE GENERER UN INCONFORT, UN PICOTEMENT OU UNE IRRITATION OCULAIRE OU CUTANE**
VERFAHREN UND VORRICHTUNG ZUR IDENTIFIZIERUNG EINES MÖGLICHERWEISE AUGEN- ODER HAUTBESCHWERDEN, REIZE ODER KRIBBELN VERURSACHENDEN MATERIALS
METHOD AND DEVICE FOR IDENTIFYING A MATERIAL LIKELY TO GENERATE EYE OR SKIN DISCOMFORT, TINGLING, OR IRRITATION

(30) Priorité: 02.09.2009 FR 0955968
(43) Date de publication de la demande: 11.07.2012
(73) Titulaire: View Point, 69410 Champagne au Mont D'or (FR)
(72) Inventeur: NEUZERET, Didier, F-01600 Sainte Euphemie (FR)
(74) Mandataire: Regimbeau
(86) Numéro de dépôt international: PCT/EP2010/062720
(87) Numéro de publication internationale: WO 2011/026841

(56) Documents cités:
- EP-A2- 1 343 014
- WO-A2-2004/006854
- JP-A- 2007 275 782
- US-B1- 7 137 970
- Katherin M. Slimak: "Avoidance response as a sublethal effect of pesticides on Lumbricus terrestris (Oligochaeta)", Soil Biology and Biochemistry, vol. 29, no. 3-4, 1 March 1997 (1997-03-01), pages 713-715, XP055151335, ISSN: 0038-0717, DOI: 10.1016/S0038-0717(96)00027-2

## Description

La présente invention concerne le domaine technique général des procédés et dispositifs permettant d'identifier si un produit est susceptible de générer un inconfort, un picotement ou une irritation oculaire ou cutané.

Ce produit est par exemple un produit pharmaceutique - tel qu'un collyre - ou un produit cosmétique - tel qu'un shampoing, une crème de soin etc. - ou tout autre produit - par exemple chimique - susceptible d'être en contact avec la peau d'un individu.

### Présentation de l'art antérieur

On connait un procédé pour estimer l'inconfort oculaire d'un produit tel qu'un produit cosmétique.

Ce procédé comprend la culture in vitro d'un modèle d'épiderme, le dépôt du produit que l'on souhaite étudier sur modèle d'épiderme cultivé, et l'observation au microscope du modèle d'épiderme comportant le produit pour caractériser l'inconfort oculaire de celui-ci.

Un tel procédé, développé en remplacement de l'expérimentation animale, présente l'inconvénient d'être long et couteux.

Par ailleurs, ce procédé ne permet pas d'obtenir des résultats satisfaisants en termes de quantification de l'inconfort oculaire du produit.

Un but de la présente invention est de proposer un procédé et un dispositif permettant une meilleure estimation de l'inconfort oculaire d'un produit, à moindre coût.

On connaît aussi l'utilisation de lombriciens pour étudier l'effet d'un produit tel qu'un pesticide ("Avoidance response as sublethal effect of pesticides on lumbricus terrestris (oligochaeta)", K. Smilak, Soil Biol. Biochem. Vol. 29, No 3/4, pp.713-715, 1997).

### Résumé de l'invention

A cet effet, l'invention propose un procédé selon la revendication 1 pour estimer si un produit ou susceptible d'être en contact avec la peau est susceptible de présenter un effet irritant et/ou un effet antalgique et/ou anesthésiant, caractérisé en ce qu'il comprend les étapes de :
- dépôt d'un produit dans un récipient,
- mise en place d'un lombricien ou d'un nématode dans le récipient,
- mesure du mouvement du lombricien dans le récipient,
- estimation de l'inconfort oculaire susceptible d'être généré par le produit en fonction de ladite mesure.

Des aspects du procédé selon l'invention sont les suivants :
o l'étape de mesure du mouvement du lombricien comprend l'acquisition d'une pluralité d'images du lombricien en utilisant des moyens d'acquisition d'image, et l'analyse des images acquises pour mesurer le mouvement du lombricien ;
o l'analyse des images acquises comprend la sélection de deux images successives acquises, la comparaison de la luminosité de chaque pixel de la première image à la luminosité du pixel correspondant dans la deuxième image, le comptage du nombre de pixels dont la luminosité a varié entre les première et deuxième images ;
o la génération d'une courbe de réponse temporelle issue du comptage précédent
o l'étape de détermination de l'inconfort oculaire comprend l'estimation de l'effet irritant du produit et/ou de l'effet antalgique et/ou anesthésiant du produit ;
o l'effet irritant et/ou antalgique du produit est estimé en fonction du nombre de pixels dont la luminosité a varié entre deux images successivement acquises ;
o si le nombre de pixels dont la luminosité a varié entre les première et deuxième images est supérieur à un seuil, alors le produit a un effet irritant ;
o si le nombre de pixels dont la luminosité a varié entre les première et deuxième images est inférieur à un seuil, alors le produit a un effet antalgique et/ou anesthésiant ;
o la détermination de l'inconfort oculaire est en outre fonction du temps ;
o le procédé comprend l'éclairage du récipient contenant le lombricien en utilisant un rayonnement infra rouge.

L'invention concerne également un produit programme d'ordinateur comportant un code programme enregistré sur un support de données lisible par un ordinateur pour exécuter le procédé décrit ci-dessus lorsque le programme d'ordinateur est appliqué à un ordinateur pour être exécuté.

### Brève description des dessins

D'autres avantages et caractéristiques ressortiront mieux de la description qui va suivre de plusieurs variantes d'exécution, données à titre d'exemples non limitatifs, à partir des dessins annexés sur lesquels :
- la figure 1 illustre un mode de réalisation du procédé selon l'invention,
- la figure 2 illustre un mode de réalisation du dispositif selon l'invention,
- la figure 3 illustre le nombre de pixels dont la luminosité a varié en fonction du temps,

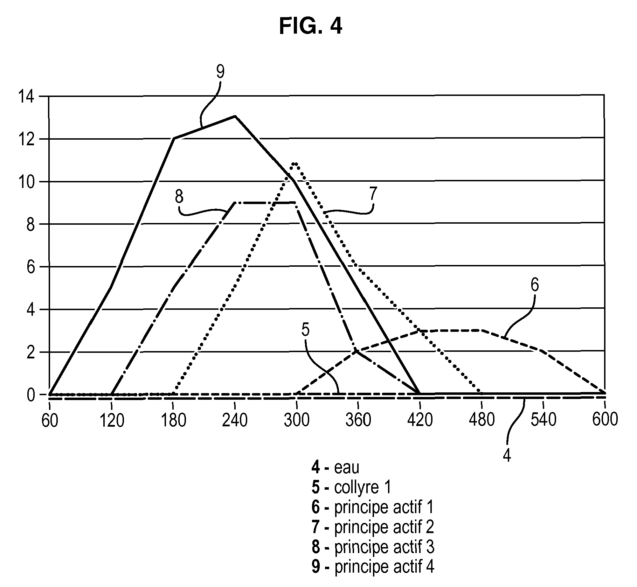

- la figure 4 illustre une représentation temporelle d'un tableau indiquant, pour un produit donné, le temps passé en hyperactivité pour une période de 60 secondes et ceci pour un total de 10 minutes.

### Description détaillée de l'invention.

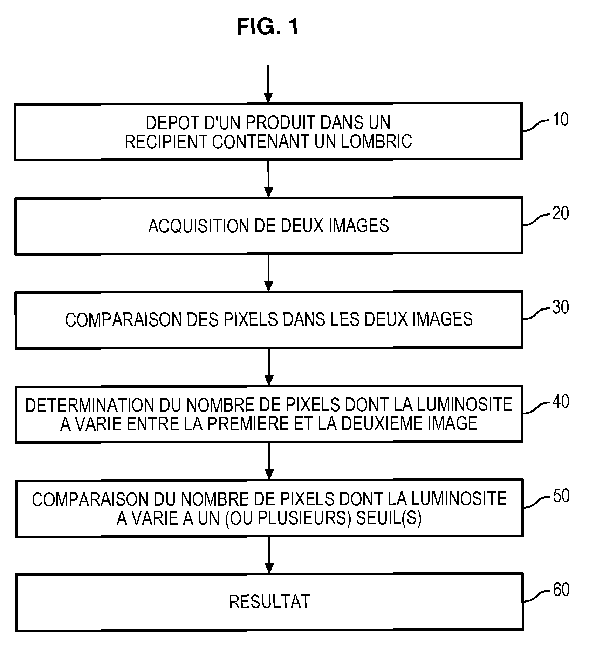

En référence à la figure 1, un mode de réalisation du procédé selon l'invention. Ce procédé permet d'identifier un produit susceptible de générer de l'inconfort, du picotement ou de l'irritation oculaire ou cutané.

Le procédé comprend les étapes de :
- dépôt 10 d'un produit dans un récipient contenant un lombricien,
- mesure 20, 30, 40 du mouvement du lombricien dans le récipient,
- estimation 50, 60 de l'inconfort oculaire susceptible d'être généré par le produit en fonction de ladite mesure.

L'avantage de l'utilisation d'un lombricien est que sa peau présente une bonne pénétration/ absorption du produit que l'on souhaite analyser.

En effet, en fonction des mouvements du lombricien dans le récipient suite au dépôt du produit, le procédé selon l'invention permet de déterminer si le produit est susceptible de générer un inconfort oculaire.

Par exemple, si les mouvements du lombricien sont très importants suite à l'introduction du produit, alors le produit présente des propriétés irritantes.

Si le lombricien ne bouge plus dans le récipient, ceci peut être lié soit à la mort de celui-ci (si le produit est très irritant notamment), soit au fait que le produit présente des propriétés antalgique, ce qui n'est pas souhaitable pour un produit tel qu'un collyre, un shampoing ou une crème.

L'utilisation d'un lombricien dans le cadre du procédé selon l'invention permet donc d'identifier si un produit est susceptible ou non de générer un inconfort oculaire ou cutané.

De préférence, le lombricien est du type « *Eisenia ».*

L'étape de mesure du mouvement du lombricien peut être réalisée en utilisant différents systèmes de mesure. Par exemple, on peut mesurer le mouvement du lombricien en utilisant un système comprenant un support muni de jauges de contrainte. Le support est destiné à recevoir le récipient contenant le lombricien. Les jauges de contraintes permettent de mesurer la variation d'assiette du support en fonction du temps, et ainsi d'estimer le mouvement du lombricien.

Dans le mode de réalisation illustré à la figure 1, l'étape de mesure du mouvement du lombricien est réalisée par analyse d'images. Le principe de la mesure du mouvement du lombricien est alors le suivant.

Une série d'images est acquise 20 par des moyens d'acquisition tout au long de l'expérience. Les moyens d'acquisition permettent l'acquisition d'images en niveaux de gris, c'est-à-dire qu'à chaque pixel d'une image correspond une valeur - représentative de la luminosité - comprise entre 0 (noir) et 255 (blanc).

Au fur et à mesure de l'acquisition (ou en variante en fin d'acquisition), les images sont comparées 30 deux à deux. Chaque image acquise au temps t est comparée à l'image successive acquise au temps t+1. Le but de cette comparaison est de déterminer le nombre les pixels dont la luminosité a varié entre les deux images.

Pour se faire, dans un mode de réalisation de l'invention, on soustrait la deuxième image à la première image. Plus précisément, on soustrait à la luminosité de chaque pixel 1 de la première image, la luminosité du pixel 2 correspondant dans la deuxième image. Dans le cas où le résultat de la différence entre deux pixels en valeur absolue est inférieur à un seuil de sensibilité, celui-ci est remis à zéro. On obtient ainsi une image résultante dans laquelle les pixels dont la luminosité est différente de zéro correspondent aux portions du lombricien ayant bougé entre les deux images. En effectuant un balayage des pixels de l'image résultante, on détermine le nombre de pixels 3 dont la luminosité a varié entre les deux images analysées. Ceci permet de caractériser le mouvement du lombricien dans le récipient entre les deux images successives analysées.

On entend, dans le cadre de la présente invention par « *pixel correspondent »,* un pixel dont les coordonnées (x2, y2) dans une deuxième image sont les mêmes que les coordonnées (x1, y1) d'un pixel dans une première image (i.e. x1 = x2 et y1 = y2).

Avantageusement, le seuil de sensibilité permet d'éliminer les bruits d'acquisition. En effet, même sans mouvement du lombricien, la luminosité de deux pixels correspondants dans les première et deuxième images peut varier du fait du bruit d'acquisition.

Une fois que l'on connaît le nombre de pixels dont la luminosité a varié entre les deux images, on détermine 50, 60 le type de réponse généré par le produit. Pour se faire, on compare 50 le nombre de pixels dont la luminosité a varié à un seuil appelé « *seuil d'hyperactivité ».* Si ce nombre de pixels est supérieur au seuil d'hyperactivité, alors le produit présente un effet irritant. Cette irritation est d'autant plus marquée que le temps passé au dessus du seuil d'hyperactivité au cours de l'expérimentation est important.

Selon l'invention, le nombre de pixels dont la luminosité a varié entre les deux images est également comparé à un autre seuil appelé « *seuil d'hypoactivité ».* Si ce nombre de pixels est inférieur au seuil d'hypoactivité, alors le produit présente un effet antalgique et/ou anesthésiant d'autant plus marqué que le temps passé au dessous du seuil d'hypoactivité au cours de l'expérimentation est important.

Avantageusement, la détermination de l'inconfort oculaire ou cutané est fonction du temps. Ceci permet une quantification de l'inconfort oculaire ou cutané associé au produit. Cette quantification de l'inconfort oculaire se caractérise d'une part par la durée d'hyperactivité (ou d'hypoactivité) du lombricien durant l'expérience, et d'autre part par le moment ou cette hyperactivité (ou hypoactivité) intervient.

Pour expliquer ce phénomène, considérons uniquement l'effet irritant d'un produit. En ce qui concerne la durée de l'hyperactivité, on comprend que plus le lombricien passe de temps en hyperactivité, plus l'inconfort (oculaire ou cutané) est important. En ce qui concerne le moment de cette hyperactivité, plus la durée entre le dépôt du produit dans le récipient et le point de départ de l'hyperactivité est faible, plus l'inconfort (oculaire ou cutané) du produit est important. A contrario, si l'hyperactivité du lombricien intervient 14 minutes après le début de l'expérience, on peut considérer que le produit a un effet irritant moindre (par rapport à un produit pour lequel le vers entre en hyperactivité dès qu'il est mis en contact avec celui-ci).

Le Tableau 1 donne une liste de résultats issus de mesure sur des produits non irritant tel eau pure ou collyre parfait suivi de principe actif dont le caractère irritant est croissant.

**TABLEAU 1**

| Temps en hyperactivité (sec) | 60 | 120 | 180 | 240 | 300 | 360 | 420 | 480 | 540 | 600 |
|---|---|---|---|---|---|---|---|---|---|---|
| eau | 0 | 0 | 0 | 0 | 0 | 0 | 0 | 0 | 0 | 0 |
| collyre1 | 0 | 0 | 0 | 0 | 0 | 0 | 0 | 0 | 0 | 0 |
| principe actif 1 | 0 | 0 | 0 | 0 | 0 | 2 | 3 | 3 | 2 | 0 |
| principe actif 2 | 0 | 0 | 0 | 5 | 11 | 6 | 3 | 0 | 0 | 0 |
| principe actif 3 | 0 | 0 | 5 | 9 | 9 | 2 | 0 | 0 | 0 | 0 |
| principe actif 4 | 0 | 5 | 12 | 13 | 10 | 5 | 0 | 0 | 0 | 0 |

Chaque ligne donne pour un produit donné le temps passé en hyperactivité pour une période de 60 secondes et ceci pour un total de 10mn.

La Courbe 1 donne une représentation temporelle de ce tableau.

Le Tableau 2 reprend la durée totale en hyperactivité sur 10 minutes des produits précédents et donne aussi la latence de la première hyperactivité.

**TABLEAU 2**

| produit | temps en hyperactivité | latence hyper actvité 1 |
|---|---|---|
| eau | 0 | 600 |
| collyre 1 | 0 | 600 |
| principe actif 1 | 10 | 320 |
| principe actif 2 | 25 | 220 |
| principe actif 3 | 25 | 160 |
| principe actif 4 | 45 | 100 |

Les principes actifs 2 et 3 présentent une durée globale équivalente mais on classifie le principe actif 3 comme plus irritant puisqu'il entraine une réponse plus rapide.

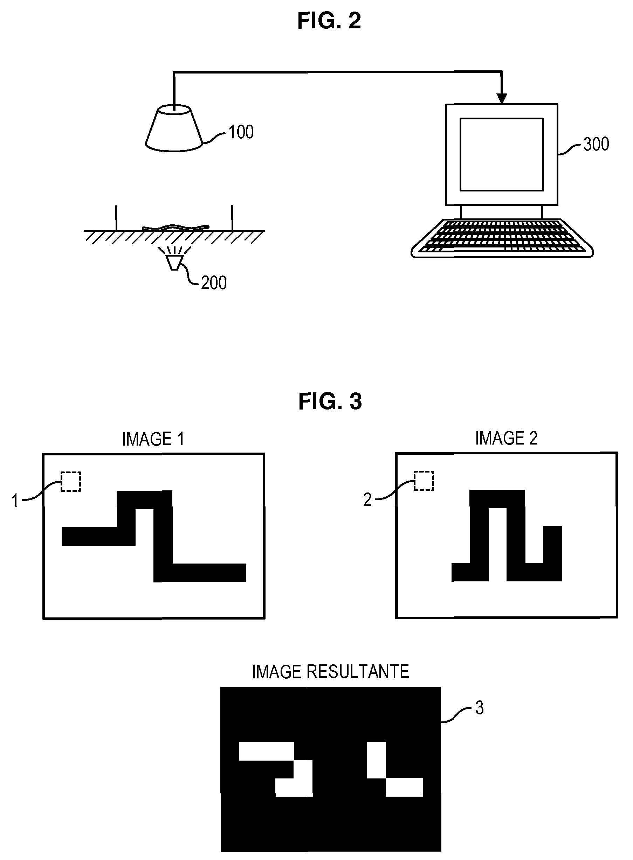

On va maintenant décrire un mode de réalisation du dispositif selon l'invention en référence à la figure 2. Le dispositif selon l'invention comprend des moyens d'acquisitions 100. Les moyens d'acquisition comprennent par exemple une caméra de type CCD. Les moyens d'acquisition 100 permettent l'acquisition d'images à une certaine fréquence (par exemple 25 images par secondes) et tout au long de l'expérience (par exemple 10 minutes).

En fonction de l'ouverture de la caméra CCD, il est possible d'acquérir une image représentant plusieurs récipients comprenant chacun un lombricien. Dans ce cas, chaque récipient peut être associé :
- à un produit respectif pour comparer l'effet irritant (ou antalgique/anesthésiant) de différents produits, ou
- au même produit dans des concentrations différentes pour comparer l'effet irritant (ou antalgique/anesthésiant) d'un produit en fonction de sa concentration.

Pour augmenter la pertinence de la comparaison on utilise des lombriciens ayant les mêmes caractéristiques - en termes d'espèce, de poids, de taille et d'âge - d'un récipient à l'autre.

Dans le cas où plusieurs récipients sont représentés dans une même image, celle-ci est subdivisée en une pluralité de régions qui sont traitées indépendamment les unes des autres, chaque région correspondant à un récipient respectif.

Les moyens d'acquisition 100 sont associés à une source lumineuse 200 pour éclairer l'objet à imager. Dans un mode de réalisation, la source lumineuse 200 comprend un négatoscope sur lequel est disposé le récipient contenant le lombricien. Ceci permet d'augmenter le contraste entre le lombricien et le récipient dans lequel il est contenu. Toutefois, le négatoscope présente l'inconvénient de chauffer. Ceci peut être gênant lors de l'analyse de produit volatil dont la concentration peut varier du fait de l'évaporation engendrée par la chaleur que génère le négatoscope. Par ailleurs, dans certains cas, l'effet irritant peut être augmenté (ou minimisé) du fait de la chaleur généré par le négatoscope. C'est pourquoi, dans un mode de réalisation, la source lumineuse est une source de lumière infrarouge disposée sous le récipient contenant le lombricien. La source de lumière infrarouge présente l'avantage de ne générer beaucoup moins de chaleur qu'un négatoscope et d'éviter de modifier des principes actifs photosensibles. Une combinaison source froide de lumières blanche et infrarouge utilisables indépendamment permet d'élargir encore le champ d'applications.

Les moyens d'acquisition 100 sont connectés à des moyens de traitement 300 pour le traitement des images acquises. Les moyens de traitement 300 comprennent par exemple un microprocesseur qui peut être intégré aux moyens d'acquisition 100, ou être distant des moyens d'acquisition 100. Dans un mode de réalisation, les moyens de traitement 300 comprennent un ordinateur de type PC.

Les moyens de traitement 300 permettent de mettre en oeuvre les étapes du procédé correspondant à la mesure 20, 30, 40 du mouvement du lombricien dans le récipient, et à la détermination 50, 60 de l'inconfort oculaire susceptible d'être généré par le produit en fonction de ladite mesure.

L'homme du métier aura compris que de nombreuses modifications peuvent être apportées au procédé et au dispositif décrits ci-dessus sans sortir matériellement des nouveaux enseignements présentés ici. Il est donc bien évident que les exemples qui viennent d'être donnés ne sont que des illustrations particulières en aucun cas limitatives.

## Revendications

1. Procédé pour estimer si un produit oculaire ou susceptible d'être en contact avec la peau est susceptible de présenter un effet irritant et/ou un effet antalgique et/ou anesthésiant, **caractérisé en ce qu'**il comprend les étapes de :
- dépôt (10) d'un produit dans un récipient,
- dépôt d'un lombricien dans le récipient,
- mesure (20, 30, 40) du mouvement du lombricien dans le récipient, comprenant :
• l'acquisition (20) d'une pluralité d'images du lombricien en utilisant des moyens d'acquisition d'image,
• l'analyse des images acquises pour mesurer le mouvement du lombricien, comprenant
o la sélection de deux images successives acquises,
o la comparaison (30) de la luminosité de chaque pixel de la première image à la luminosité du pixel correspondant dans la deuxième image,
o le comptage du nombre de pixels dont la luminosité a varié entre les première et deuxième images,
- comparaison du nombre de pixels dont la luminosité a varié entre la première image et la deuxième image avec un seuil, et
- estimation que ledit produit est susceptible de présenter un effet irritant si le nombre de pixels dont la luminosité à varié est supérieur à un seuil dit seuil d'hyperactivité, et/ou
- estimation que ledit produit est susceptible de présenter un effet antalgique et/ou anesthésiant si le nombre de pixels dont la luminosité à varié est inférieur à un seuil dit seuil d'hypoactivité.

2. Procédé selon la revendication précédente, dans lequel l'effet du produit est quantifié en outre en fonction du temps pendant lequel nombre de pixels dont la luminosité à varié est au-dessus du seuil d'hyperactivité ou en dessous du seuil d'hypoactivité.

3. Procédé selon l'une des revendications précédentes, **caractérisé en ce qu'**il comprend l'éclairage du récipient contenant le lombricien en utilisant un rayonnement infra rouge disposé sous le récipient contenant le lombricien.

4. Produit programme d'ordinateur comportant un code programme enregistré sur un support de données lisible par un ordinateur pour exécuter le procédé selon l'une des revendications 1 à 3 lorsque le programme d'ordinateur est appliqué à un ordinateur pour être exécuté.

## Patentansprüche

1. Verfahren, um festzustellen, ob ein Produkt für die Augen oder welches mit der Haut in Kontakt kommen könnte, eine reizende Wirkung und/oder eine schmerzstillende und/oder anästhesierende Wirkung haben könnte, **dadurch gekennzeichnet, dass** es die folgenden Schritte umfasst:
- Verbringen (10) eines Produkts in einen Behälter,
- Verbringen eines Wurms in den Behälter,
- Messen (20, 30, 40) der Bewegung des Wurms in dem Behälter, umfassend:
• die Aufnahme (20) einer Vielzahl von Bildern des Wurms anhand der Benutzung von Bildaufnahmemitteln,
• die Analyse der aufgenommenen Bilder, um die Bewegung des Wurms zu messen, umfassend
o die Auswahl von zwei aufeinanderfolgend aufgenommenen Bildern,
o den Vergleich (30) der Lichtstärke jedes Pixels des ersten Bilds mit der Lichtstärke des entsprechenden Pixels des zweiten Bilds,
o das Zählen der Anzahl der Pixel, deren Lichtstärke sich zwischen dem ersten und zweiten Bild verändert hat,
- Vergleichen der Anzahl der Pixel, deren Lichtstärke sich zwischen dem ersten Bild und dem zweiten Bild mit einem Grenzwert verändert hat, und
- Feststellen, dass das Produkt eine reizende Wirkung haben könnte, wenn die Anzahl der Pixel, deren Lichtstärke sich verändert hat, über einem Hyperaktivitätsgrenzwert liegt, und/oder
- Feststellen, dass das Produkt eine schmerzstillende und/oder anästhesierende Wirkung haben könnte, wenn die Anzahl der Pixel, deren Lichtstärke sich verändert hat, unter einem Hyperaktivitätsgrenzwert liegt.

2. Verfahren nach vorangehendem Anspruch, wobei die Wirkung des Produkts ferner in Abhängigkeit von der Zeit quantifiziert wird, während der die Anzahl der Pixel, deren Lichtstärke sich verändert hat, über dem Hyperaktivitätsgrenzwert oder unter dem Hyperaktivitätsgrenzwert ist.

3. Verfahren nach einem der vorangehenden Ansprüche, **dadurch gekennzeichnet, dass** es die Beleuchtung des den Wurm enthaltenden Behälters mit einer Infrarotstrahlung umfasst, die unter dem den Wurm enthaltenden Behälter angeordnet ist.

4. Rechnerprogrammprodukt, umfassend einen Programmcode, der auf einem rechnerlesbaren Datenträger gespeichert ist, um das Verfahren nach einem der Ansprüche 1 bis 3 auszuführen, wenn das Rechnerprogramm zwecks seiner Ausführung auf einem Rechner angewendet wird.

## Claims

1. A method for identifying whether an eye product or product which may be in contact with the skin is likely to have an irritating effect and/or an antalgic and/or anesthetic effect, **characterized in that** it comprises the steps of:
- disposition (10) of a product in a container,
- disposition of an earthworm in the container,
- measurement (20, 30, 40) of the movement of the earthworm in the container, comprising:
• acquisition (20) of a plurality of images of the earthworm by using image acquisition means,
• analysis of the acquired images for measuring the movement of the earthworm, comprising
o selection of two acquired successive images,
o comparison (30) of the luminosity of each pixel of the first image with the luminosity of the corresponding pixel in the second image,
o counting the number of pixels for which the luminosity has changed between the first and second images,
- comparison of the number of pixels for which the luminosity has changed between the first image and the second image with a threshold, and
- identifying whether said product is likely to have an irritating effect if the number of pixels, for which the luminosity has changed, is greater than a threshold, a so-called hyperactivity threshold, and/or
- identifying whether said product is likely to have an antalgic and/or anesthetic effect if the number of pixels, for which the luminosity has changed, is less than a threshold, a so-called hypoactivity threshold.

2. The method according to the preceding claim, wherein the effect of the product is further quantified according to the period during which the number of pixels, for which the luminosity has changed, is above the hyperactivity threshold or below the hypoactivity threshold.

3. The method according to one of the preceding claims, **characterized in that** it comprises the illumination of the container containing the earthworm by using infrared radiation positioned under the container containing the earthworm.

4. A computer program product including program code recorded on a data medium legible by a computer for executing the method according to one of claims 1 to 3 when the computer program is applied to a computer in order to be executed.
